# EUROPÄISCHE PATENTANMELDUNG

(11) **EP 1 433 785 A1**
(43) Veröffentlichungstag der Anmeldung: **30.06.2004**
(21) Anmeldenummer: 03028795.7
(22) Anmeldetag: 13.12.2003
(51) Int. Cl.: C07D 215/12, C07D 213/53, A61K 7/13, C07D 401/12, C07D 417/12, C09B 55/00

(54) **Mittel zum Färben von keratinhaltigen Fasern**

(30) Priorität: 23.12.2002 DE 10260879
(71) Anmelder: Henkel Kommanditgesellschaft auf Aktien, 40589 Düsseldorf (DE)
(72) Erfinder: Möller, Hinrich, Dr., 40789 Monheim (DE); Höffkes, Horst, Dr., 40595 Düsseldorf (DE); Oberkobusch, Doris, Dr., 40591 Düsseldorf (DE)

(57) **Zusammenfassung**

Es werden Azomethine mit der Formel I beansprucht 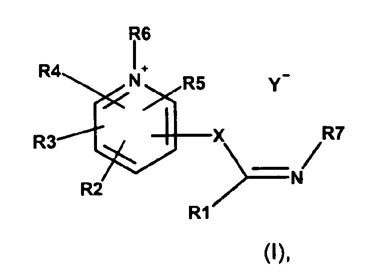 in der R¹, R², R³, R⁴, R⁵, R⁶, R⁷, X und Y⁻ die in den Patentansprüchen und der Beschreibung definierte Bedeutung haben.
Die beanspruchten Azomethine eignen sich insbesondere als färbende Komponente in Mitteln zum Färben von keratinhaltigen Fasern, insbesondere zum Färben von menschlichen Haaren.

## Beschreibung

Die Erfindung betrifft Azomethine, ein Verfahren zur Herstellung von Azomethinen, die Verwendung der Azomethine als färbende Komponente in Mitteln zum Färben von keratinhaltigen Fasern, ein Mittel zum Färben von keratinhaltigen Fasern, insbesondere menschlichen Haaren, das Azomethine enthält, sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Die Verwendung der unten näher beschriebenen Azomethine zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass Azomethine mit der Formel I sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der vorliegenden Erfindung sind Azomethine mit der Formel I 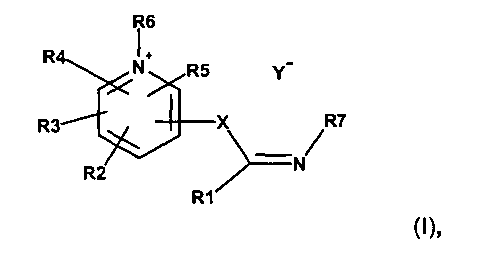 in der
- R¹ steht für ein Wasserstoffatom, eine C₁₋₄-Alkylgruppe, eine Arylgruppe oder eine Heteroarylgruppe,
- R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
   wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
- R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
- R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
   oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
- X steht für eine direkte Bindung, eine gegebenenfalls substituierte Vinylen- oder Phenylengruppe, die sich auch an einem ankondensierten aromatischen Ring befinden kann, und
- Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet.

Beispiele für die als Substituenten im Rahmen dieser Anmeldung genannten C₁₋₄-Alkylreste sind die Gruppen Methyl, Ethyl, Propyl, Isopropyl, n-Butyl, sec-Butyl und tert.-Butyl. Ethyl und Methyl sind bevorzugte Alkylreste. Bevorzugte C₃₋₆-Cycloalkylgruppen sind die Cyclobutylgruppe, die Cyclopropylgruppe und die Cyclohexylgruppe. Erfindungsgemäß bevorzugte C₁₋₄-Alkoxyreste sind beispielsweise eine Methoxy- oder eine Ethoxygruppe. Weiterhin können als bevorzugte Beispiele für eine C₁₋₄-Hydroxyalkylgruppe eine Hydroxymethyl-, eine 2-Hydroxyethyl-, eine 2-Hydroxypropyl, eine 3-Hydroxypropyl- und eine 4-Hydroxybutylgruppe genannt werden. Eine 2-Hydroxyethylgruppe ist besonders bevorzugt. Beispiele für eine C₂-C₆-Dihydroxyalkylgruppe ist die 2,3-Dihydroxypropylgruppe. Eine bevorzugte Hydroxy-C₁₋₄-Alkoxygruppe ist die 2-Hydroxyethoxygruppe. Bevorzugte Arylgruppen sind Phenyl, Naphthyl und Biphenyl. Beispiele für Halogenatome sind F-, Cl-, oder Br-Atome, wobei Cl-Atome ganz besonders bevorzugt sind. Bevorzugte C₁₋₄-Aminoalkylgruppen sind die Aminomethyl-, die Aminoethyl und die Aminopropylgruppe. Bevorzugte C₁₋₄-Acylgruppen sind Formyl, Acetyl, Propionyl und Butyryl. Eine bevorzugte C₂₋₄-Carboxyalkylgruppe ist die Carboxymethylgruppe. Die Sulfomethylgruppe, die 2-Sulfoethylgruppe, die 2-Sulfopropylgruppe und die 3-Sulfopropylgruppe sind bevorzugte C₁₋₄-Sulfoalkylgruppen. Beispiele für bevorzugte C₁₋₄-Sulfonatoalkylgruppen sind die Sulfonatomethylgruppe, die 2-Sulfonatoethylgruppe, die 2-Sulfonatopropylgruppe und die 3-Sulfonatopropylgruppe. Beispiele für bevorzugte C₁₋₄-Carboxylatoalkylgruppen sind die Carboxylatomethyl- und die 2-Carboxylatoethylgruppe. Beispiele für eine Aryl-C₁₋₄-alkylgruppe sind Benzyl und 2-Phenylethyl. Beispiele für eine Heteroarylgruppe sind Pyrrolidyl, 2-Furyl, 2-Thienyl, 4-Pyridyl, 3-Pyridyl, 2-Pyridyl, Triazolyl, Tetrazolyl, Pyrazolyl, Benzothiazolyl, Benzoxazolyl, Pyrimidyl, Imidazolinyl und Imidazolyl. Die weiteren verwendeten Begriffe leiten sich erfindungsgemäß von den hier gegebenen Definitionen ab. Die oben genannten Beispiele der verwendeten Reste sind somit auch für die folgenden Formeln II bis V relevant.

Die erfindungsgemäßen Verbindungen eignen sich insbesondere zum Färben von keratinhaltigen Fasern.

Bevorzugte Verbindungen mit der Formel I sind 2-Cyclohexyliminomethyl-1-methylpyridinium-, 3-Cyclohexyliminomethyl-1-methylpyridinium-, 4-Cyclohexyliminomethyl-1-methylpyridinium-, 4-Benzyliminomethyl-1-methylpyridinium-, 4-(4-Hydroxybenzyliminomethyl)-1-methylpyridinium-, 4-(4-Hydroxyphenyliminomethyl)-1-methylpyridinium-, 4-(4-Carboxyphenyliminomethyl)-1-methylpyridinium-, 2-(4-Carboxyphenyliminomethyl)-1-ethylpyridinium-, 3-(4-Carboxyphenyliminomethyl)-1-ethylpyridinium-, 4-(4-Carboxyphenyliminomethyl)-1-ethylpyridinium-, 4-(2-Hydroxyethyliminomethyl)-1-methylpyridinium-, 4-(2-Hydroxypropyliminomethyl)-1-methylpyridinium-, 4-(Carboxymethyliminomethyl)-1-methylpyridinium-, 2-(2-Carboxyethyliminomethyl)-1-ethylpyridinium-, 3-(2-Carboxyethyliminomethyl)-1-ethylpyridinium-, 4-(2-Carboxyethyliminomethyl)-1-ethylpyridinium-, 1-Benzyl-2-(4-sulfophenyliminomethyl)-pyridinium-, 1-Benzyl-4-(4-sulfophenyliminomethyl)-pyridinium-, 4-(4-Carboxyphenyliminomethyl)-1-methylpyridinium-, 2-(4-Carboxyphenyliminomethyl)-1-methylchinolinium-, 4-(4-Carboxyphenyliminomethyl)-1-methylchinolinium-, 5-(4-Carboxyphenyliminomethyl)-1-methylchinolinium-, 7-(4-Carboxyphenyliminomethyl)-1-methylchinolinium-, 2-(4-Carboxy-3-hydroxyphenyliminomethyl)-1-methylchinolinium-, 4-(4-Carboxy-3-hydroxyphenyliminomethyl)-1-methylchinolinium-, 2-(4-Cyanphenyliminomethyl)-1-methylchinolinium-, 4-(4-Cyanphenyliminomethyl)-1-methylchinolinium-, 2-(2,4-Dihydroxy-6-pyrimidyliminomethyl)-1-methylchinolinium-, 4-(2,4-Dihydroxy-6-pyrimidyliminomethyl)-1-methylchinolinium-, 4-(2,4-Dihydroxy-6-pyrimidyliminomethyl)-1-methylpyridinium-, 4-(Benzothiazol-2-yliminomethyl)-1-methylchinolinium-halogenid, wie -chlorid, -bromid oder -iodid, -benzolsulfonat, p-toluolsulfonat, C₁₋₄-alkansulfonat, wie -methansulfonat oder -ethansulfonat, -trifluormethansulfonat, -perchlorat, 0.5 -sulfat, -hydrogensulfat, -tetrafluoroborat, -phosphat, -hexafluorophosphat oder -tetrachlorozinkat.

Die Azomethine mit der Formel I werden in üblicher Weise durch Kondensation der entsprechenden Carbonylverbindungen mit geeigneten primären Aminen hergestellt.

Ein weiterer Gegenstand der vorliegenden Erfindung ist ein Verfahren zur Herstellung von Azomethinen mit der Formel I 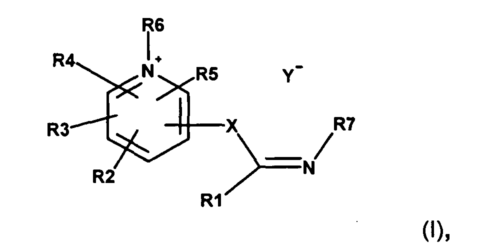 in der R¹, R², R³ R⁴, R⁵, R⁶, R⁷, X und Y⁻ wie oben definiert sind,
worin eine Carbonylverbindung mit der Formel II 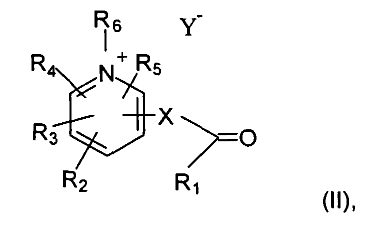 in der R¹, R², R³, R⁴, R⁵, R⁶, X und Y⁻ wie oben definiert sind,
und ein Amin mit der Formel III

H₂N-R⁷ (III),

in der R⁷ wie oben definiert ist,
in an sich bekannter Weise einer Kondensationsreaktion unterworfen werden.

Die Kondensationsreaktion wird üblicherweise in Gegenwart eines Lösungsmittels, wie C₁₋₄-Alkanolen, Ethern, Kohlenwasserstoffen, insbesondere cyclischen Kohlenwasserstoffen, wie Cyclohexan und Toluol, chlorierten Kohlenwasserstoffen, wie Methylenchlorid, Chloroform oder Tetrachlorkohlenstoff, bei Raumtemperatur oder erhöhten Temperaturen, insbesondere zwischen 50°C und 150°C durchgeführt. Das sich bei der Reaktion bildende Wasser kann azeotrop entfernt werden.

Bevorzugte Verbindungen mit der Formel II sind die Benzolsulfonate, p-Toluolsulfonate, Methansulfonate, Perchlorate, Sulfate, Chloride, Bromide, Iodide, Tetrachlorozinkate von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 4-(2-Formylvinyl)-1-methylchinolium, 4-Acetyl-1-methylpyridinium, 2-Acetyl-1-methylpyridinium, 4-Acetyl-1-methylchinolin und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 4-Pyridinaldehyd-N-oxid und N-Methylpyridoxal,
2- und 4-Formyl-1-methylchinolinium-p-toluolsulfonat, Acridin-1-carboxaldehyd, Acridin-2-carboxaldehyd, Acridin-3-carboxaldehyd, Acridin-4-carboxaldehyd, Acridin-9-carboxaldehyd, Benz[c]acridin-7-carboxaldehyd, 1-Formyl-10-methyl-acridinium-, 2-Formyl-10-methyl-acridinium-, 3-Formyl-10-methyl-acridinium-, 4-Formyl-10-methyl-acridinium-, 9-Formyl-10-methyl-acridinium-, 9-Formyl-2,10-dimethylacridinium-, 9-Formyl-10-ethyl-acridinium-, 9-Formyl-2-chlor-10-methyl-acridinium-, 9-Formyl-2-methoxy-10-methyl-acridinium-, 9-Formyl-2-chlor-10-methyl-acridinium-, 9-Formyl-3,6-dichlor-10-methyl-acridinium-, 9-Acetyl-10-methyl-acridinium-, 9-Benzolyl-10-methyl-acridinium-iodid, -bromid, -methylsulfat, -ethylsulfat -p-toluolsulfonat, -trifluormethansulfonat, -methansulfonat, -tetrafluorborat, 9-Formylacridin-N-oxid, 7-Formyl-10-methylbenz[c]acridinium-methylsulfat,
Chinoliniumaldehyde und ―ketone, insbesondere die Benzolsulfonate, Tetrafluoroborate, p-Toluolsulfonate, Methansulfonate, Trifluormethansulfonate, Perchlorate, Sulfate, Chloride, Bromide, Iodide, Tetrafluoroborate und/oder Tetrachlorozinkate von 5-Formyl-1-methyl-chinolinium, 6-Formyl-1-methyl-chinolinium, 7-Formyl-1-methyl-chinolinium, 8-Formyl-1-methyl-chinolinium, 5-Formyl-1-ethyl-chinolinium, 6-Formyl-1-ethyl-chinolinium, 7-Formyl-1-ethyl-chinolinium, 8-Formyl-1-ethyl-chinolinium, 5-Formyl-1-benzylchinolinium, 6-Formyl-1-benzyl-chinolinium, 7-Formyl-1-benzyl-chinolinium, 8-Formyl-1-benzyl-chinolinium, 5-Formyl-1-allyl-chinolinium, 6-Formyl-1-allyl-chinolinium, 7-Formyl-1-allyl-chinolinium, 8-Formyl-1-allylchinolinium, 5-Acetyl-1-methyl-chinolinium, 6-Acetyl-1-methyl-chinolinium, 7-Acetyl-1-methyl-chinolinium, 8-Formyl-1-Acetyl-chinolinium, 9-Formyl-10-methylacridinium sowie beliebige Gemische der voranstehenden.

Bevorzugte Beispiele für die als Verbindungen mit der Formel III eingesetzten Amine sind 2-Hydroxyethylamin, n-Butylamin, 2-Butylamin, 2-Hydroxypropylamin, 3-Hydroxypropylamin, n-Hexylamin, Cyclohexylamin, 2,3-Dihydroxyalkylamin, 2-Aminoethansulfonsäure, α-Aminosäuren, wie Glycin, Alanin, Serin, Threonin, Lysin, Glutaminsäure, Asparaginsäure, Prolin, Tryptophan, Histidin, Arginin, Phenylalanin, 4-Hydoxyphenylalanin, β-Alanin, 6-Aminocapronsäure, Anilin, Chlor- und Brom-substitierte Aniline, Anthranilsäure, 3-Aminobenzoesäure, 4-Aminobenzoesäure, Sulfanilsäure, 4-Aminosalicysäure, 5-Aminosalicysäure, Melamin, 2-Aminobenzonitril, 3-Aminobenzonitril, 4-Aminobenzonitril, 2-Aminophenol, 3-Aminophenol, 4-Aminophenol, 4-Amino-2-aminomethylphenol, 2-Methoxyanilin, 3-Methoxyanilin, 4-Methoxyanilin, 2-Methylanilin, 3-Methylanilin, 4-Methylanilin, 4-Amino-2-methylphenol, 5-Amino-2-methylphenol, 4-Amino-3-methylphenol, 5-Amino-3-methylphenol, 4-Aminouracil, 6-Aminouracil, 5-Aminouracil, 2-Aminopyridin, 3-Aminopyridin, 4-Aminopyridin, 2-Amino-3-hydroxypyridin, 2,6-Diaminopyridin, 2,5-Diaminopyridin, 2,3-Diaminopyridin, 2-Dimethylamino-5-aminopyridin, 3-Amino-2-methylamino-6-methoxypyridin, 2,3-Diamino-6-methoxypyridin, 2,4,5-Triaminopyridin, 2,6-Dihydroxy-3,4-dimethylpyridin, 2-Hydroxy-3-aminopyridin, 2-Hydroxy-4-aminopyridin, 3,5-Diamino-2,6-dimethoxypyridin, 4,5,6-Triaminopyrimidin, 4-Hydroxy-2,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diamino-pyrazol, 3,5-Diamino-1,2,4-triazol, 3-Amino-1,2,4-triazol, 2-Aminothiazol, 5-Aminotetrazol, 3-Aminopyrazol, 3-Amino-5-hydroxypyrazol, 1-Phenyl-3-carbamoyl-4-amino-5-pyrazolon, 1-Phenyl-4,5-diamino-pyrazol, 1-Phenyl-3-methyl-4,5-diaminopyrazol, 1-(2-Hydroxyethyl)-4,5-diamino-pyrazol. 2-Aminoimidazol, 2-Aminoimidazolin, 2-Aminobenzimidazol, 2-Aminobenzothiazol, 2-Aminobenzoxazol und/oder Sulfanilamid sowie beliebige Gemische der voranstehenden.

Ein weiterer Gegenstand der vorliegenden Erfindung ist die Verwendung von Azomethinen mit der Formel I zum Färben von keratinhaltigen Fasern, insbesondere als eine färbende Komponente in Haarfärbemitteln. Dabei können die Azomethine der Formel I sowohl in direktziehenden Färbemitteln, als auch in Oxidationshaarfärbemitteln zur Anwendung kommen.

Unter keratinhaltigen Fasern sind Wolle, Pelze, Federn und insbesondere menschliche Haare zu verstehen. Die erfindungsgemäßen Azomethine können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Ein weiterer Gegenstand der vorliegenden Erfindung ist ein Mittel zum Färben von keratinhaltigen Fasern, enthaltend mindestens ein Azomethine mit der Formel I 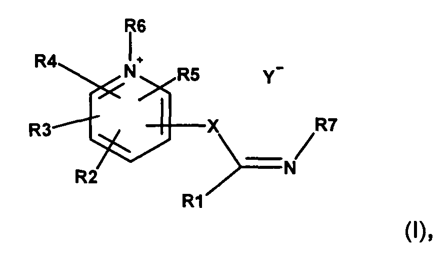 in der R¹, R², R³ R⁴, R⁵, R⁶, R⁷, X und Y⁻ wie oben definiert sind.

Die voranstehend genannten Verbindungen mit der Formel I werden vorzugsweise in den erfindungsgemäßen Mitteln zum Färben von keratinhaltigen Fasern jeweils in einer Menge von 0,03 bis 65 mmol, insbesondere von 1 bis 40 mmol, bezogen auf 100 g des gesamten Färbemittels, verwendet.

Die erfindungsgemäßen Azomethine können als direktziehende Färbemittel und auch in Gegenwart von üblichen Oxidationsfarbstoffvorprodukten, wie üblichen Kuppler- und Entwicklerkomponenten, die bereits im einleitenden Teil der Beschreibung genannt wurden, eingesetzt werden.

Färbemittel, die als färbende Komponente die erfindungsgemäßen Azomethine allein enthalten, werden bevorzugt für Färbungen im Bereich von gelb, gelbbraun, orange, braunorange, mittelbraun, dunkelbraun, violett, dunkelviolett bis hin zu blauschwarz eingesetzt.

Färbungen mit noch erhöhter Brillanz und verbesserten Echtheitseigenschaften (Lichtechtheit, Waschechtheit, Reibechtheit) über einen weiten Nuancenbereich werden erzielt, wenn die erfindungsgemäß eingesetzten Verbindungen mit der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, sowie CH-aktiven Verbindungen und quartären Ammoniumverbindungen verwendet werden. Dies sind einerseits Verbindungen, die für sich alleine keratinhaltige Fasern nur schwach färben und erst gemeinsam mit den Verbindungen der Formel I brillante Färbungen ergeben. Andererseits sind darunter aber auch Verbindungen, die bereits als Oxidationsfarbstoffvorprodukte eingesetzt werden.

In allen Färbemitteln können auch mehrere verschiedene Azomethine der Formel I gemeinsam zum Einsatz kommen; ebenso können auch mehrere verschiedene Verbindungen der Komponente B gemeinsam verwendet werden.

Die als Beispiele für Komponente B einsetzbaren Verbindungen stellen vorzugsweise Nucleophile dar. Besonders bevorzugt werden solche Verbindungen eingesetzt, die eine geringere Nucleophilie als die Verbindung mit der Formel I aufweisen.

Unter die voranstehend beschriebene Ausführungsform fällt auch die Verwendung von solchen Substanzen, die Reaktionsprodukte von Azomethinen der Formel I mit den genannten Verbindungen der Komponente B darstellen, als direktziehende Färbemittel.

Geeignete Verbindungen mit primärer oder sekundärer Aminogruppe als Komponente B sind z. B. primäre und sekundäre aromatische Amine wie N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel IV dargestellt sind 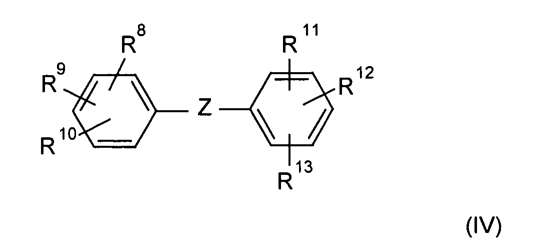 in der
- R⁸ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁹, R¹⁰, R¹¹, R¹² und R¹³ unabhängig voneinander für ein Wasserstoffatom, eine Hydroxy- oder eine Aminogruppe, die durch C₁-C₄-Alkyl-, C₁-C₄-Hydroxyalkyl, C₁-C₄-Alkoxy-, C₁-C₄-Aminoalkyl- oder C₁-C₄-Alkoxy-C₁-C₄-alkylgruppen substituiert sein kann, stehen, und
- Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel V 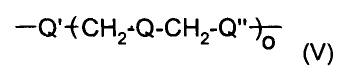 in der
- Q eine direkte Bindung, eine CH₂- oder CHOH-Gruppe bedeutet,
- Q' und Q" unabhängig voneinander für ein Sauerstoffatom, eine NR¹⁴-Gruppe, worin R¹⁴ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)_{p'}-O, worin p und p' 2 oder 3 sind, stehen und
- o eine ganze Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Als CH-acide Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolinhydrochlorid, 3-Cyan-1,4-dimethyl-6-hydroxy-2-pyridon und 3-Cyan-1-ethyl-6-hydroxy-4-methyl-2-pyridon.

Beispiele für quartäre Ammoniumsalze sind Tetramethyl-, Tetraethyl-, Tetrapropyl-, Tetrabutyl-, Benzyltrimethylammonium-chlorid, -bromid, -iodid, -hydrogensulfat, 0.5 - sulfat sowie den quaternierten Hydroxyethylcellulose-Derivaten (INCI-Bezeichnung: Polyquaternium 10).

Die Verbindungen der Komponente B werden besonders bevorzugt ausgewählt aus der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat, sowie den vorzugsweise mit anorganischen Säuren gebildeten physiologisch verträglichen Salzen der vorgenannten Verbindungen.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In einer weiteren Ausführungsform kann die Bandbreite des Nuancenbereichs der Ausfärbungen durch die Verwendung von reaktiven Carbonylverbindungen als zusätzliche Komponente C erweitert werden. Beispiele für geeignete reaktive Carbonylverbindungen sind Aldehyde oder cyclische oder nichtcyclische Ketone. Diese Verbindungen können mit den Aminoverbindungen bzw. CH-aktiven Verbindungen der Komponente B reagieren. Durch geeignete Kombination der Verbindungen mit der Formel I, der Komponente B und ggf. C läßt sich eine große Vielfalt an Ausfärbungen erhalten. Unter diese Ausführungsform fällt auch die Verwendung von solchen Substanzen, die Reaktionsprodukte von Azomethinderivaten der Formel I mit den genannten Verbindungen der Komponente B und Verbindungen der Komponente C darstellen, als direktziehende Färbemittel.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Verbindungen mit der Formel I zum Einsatz kommen; ebenso können auch mehrere verschiedene Farbverstärker gemeinsam verwendet werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkail- und Erdalkalimetalle oder aus Iodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze oder Metalloxide, die einen leichten Wechsel zwischen zwei Oxidationsstufen der Metallionen ermöglichen. Beispiele sind Salze oder Oxide von Eisen, Ruthenium, Mangan und Kupfer.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethylamino)-benzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die erfindungsgemäß enthaltenen Verbindungen mit der Formel I oder die fakultativ enthaltenen Verbindungen der Komponenten B und C sowie die Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine - COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Ammonium, Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 12, vorzugsweise zwischen 4 und 10.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend mindestens ein Azomethin mit der Formel I 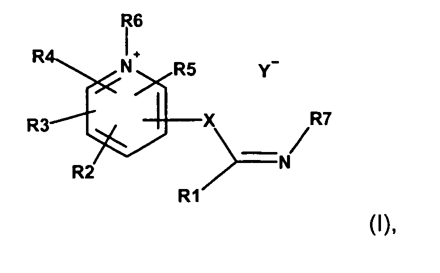 in der R¹, R², R³, R⁴, R⁵, R⁶, R⁷, X und Y⁻ wie oben definiert sind,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Azomethine der Formel I und die gegebenenfalls eingesetzten Verbindungen der Komponenten B und C sowie die ebenfalls optional einsetzbaren Farbverstärker können entweder gleichzeitig oder aber auch zeitlich voneinander getrennt in beliebiger Reihenfolge auf das Haar aufgebracht werden. Ausfärbungen mit besonders guten Echtheitseigenschaften können erhalten werden, wenn zumindest Verbindungen der Komponente B eingesetzt werden, die zuerst aufgebracht werden. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit einer Lösung der Salze ist möglich.

Die Azomethine der Formel I und die gegebenenfalls eingesetzten Farbverstärker, Oxidationsfarbstoffvorprodukte und/oder reaktiven Carbonylverbindungen können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### 1. Herstellungsbeispiele

A. 4-(4-Carboxyphenyliminomethyl)-1-methylchinolinium-p-toluolsulfonat
   Die gerührte Mischung von 17,2 g (0,05 Mol) 4-Formyl-1-methylchinolinium-p-toluolsulfonat, 6,8 g (0,05 Mol) 4-Aminobenzoesäure, 7,5 ml (0,15 Mol) und 100 ml Ethanol wurde zum Sieden erhitzt und noch 2 Std. bei 70 °C gerührt. Anschließend wurde die erhaltene Lösung stark eingeengt. Nach dem Abkühlen auf Raumtemperatur wurden die ausgefallenen Kristalle abgesaugt, mit wenig kaltem Ethanol gewaschen und getrocknet. Es wurden 21,8 g (91 % d. Th.) 4-(4-Carboxyphenyliminomethyl)-1-methylchinolinium-p-toluolsulfonat vom Schmp. 80-82 °C erhalten. Das 1H-NMR-Spektrum bestätigt die Struktur.
   Analog wurden die folgenden Azomethine synthetisiert:
B. 2-(4-Carboxyphenyliminomethyl)-1-methylchinolinium-p-toluolsulfonat: Schmp. 72-75 °C (hygrokopisch)
C. 4-(2,4-Dihydroxy-6-pyrimidyliminomethyl)-1-methylchinolinium-p-toluolsulfonat: Schmp. 260 °C.

### 2. Herstellung einer Färbelösung

Zur Herstellung der Färbelösung wurde die unter 1. hergestellten Verbindungen 1A, 1B und 1C eingesetzt.

Es wurde je eine Aufschlämmung/Lösung von 5 mmol eines Azomethins mit der Formel I und 5 mmol einer Verbindung der Komponente B in 25 ml Wasser bei 50°C hergestellt. Nach dem Abkühlen auf 30°C wurden beide Teile innig miteinander vermischt, mit 5 mmol Natriumacetat und einem Tropfen einer 20-%igen Fettalkylethersulfat-Lösung versetzt. Abschließend wurde der pH-Wert mit verdünnter Salzsäure auf pH 6 eingestellt.

Bei Ausfärbungen ohne Komponente B wurde das Azomethin in 50 ml H₂O eingetragen.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in den nachfolgenden Tabellen wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

| **Ausfärbungen** | | | |
|---|---|---|---|
| **Azomethin** | **Komponente B** | **Farbe** | **Intensität** |
| 1A | - | rotviolett | + |
| 1A | 3-Amino-2-methylamino-6-methoxypyridin | blauschwarz | +++ |
| 1A | 2-Aminomethyl-4-aminophenol x 2 HCl | gelbbraun | ++ |
| 1A | N,N-Bis-(2-hydroxyethyl)-p-phenylendiamin x H₂SO₄ | dunkelblau | ++(+) |
| 1B | - | dunkelviolett | ++(+) |
| 1B | 3-Amino-2-methylamino-6-methoxypyridin | schwarz | +++ |
| 1B | 2-Aminomethyl-4-aminophenol x 2 HCl | gelbbraun | ++(+) |
| 1B | N,N-Bis-(2-hydroxyethyl)-p-phenylendiamin x H₂SO₄ | leuchtend blau | ++(+) |
| 1C | 3-Amino-2-methylamino-6-methoxypyridin | gelbbraun | ++ |
| 1C | 2-Aminomethyl-4-aminophenol x 2 HCl | braungelb | + |
| 1C | N,N-Bis-(2-hydroxyethyl)-p-phenylendiamin x H₂SO₄ | blaugrau | ++ |

## Patentansprüche

1. Azomethine mit der Formel I 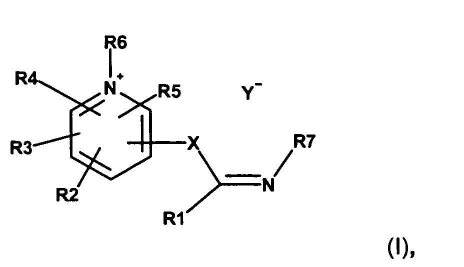 in der
• R¹ steht für ein Wasserstoffatom, eine C₁₋₄-Alkylgruppe, eine Arylgruppe oder eine Heteroarylgruppe,
• R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
• R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
• R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
• X steht für eine direkte Bindung, eine gegebenenfalls substituierte Vinylen- oder Phenylengruppe, die sich auch an einem ankondensierten aromatischen Ring befinden kann, und
• Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet.

2. Azomethine nach Anspruch 1, **dadurch gekennzeichnet, dass** sie ausgewählt sind aus 2-Cyclohexyliminomethyl-1-methylpyridinium-, 3-Cyclohexyliminomethyl-1-methylpyridinium-, 4-Cyclohexyliminomethyl-1-methylpyridinium-, 4-Benzyliminomethyl-1-methylpyridinium-, 4-(4-Hydroxybenzyliminomethyl)-1-methylpyridinium-, 4-(4-Hydroxyphenyliminomethyl)-1-methylpyridinium-, 4-(4-Carboxyphenyliminomethyl)-1-methylpyridinium-, 2-(4-Carboxyphenyliminomethyl)-1-ethylpyridinium-, 3-(4-Carboxyphenyliminomethyl)-1-ethylpyridinium-, 4-(4-Carboxyphenyliminomethyl)-1-ethylpyridinium-, 4-(2-Hydroxyethyliminomethyl)-1-methylpyridinium-, 4-(2-Hydroxypropyliminomethyl)-1-methylpyridinium-, 4-(Carboxymethyliminomethyl)-1-methylpyridinium-, 2-(2-Carboxyethyliminomethyl)-1-ethylpyridinium-, 3-(2-Carboxyethyliminomethyl)-1-ethylpyridinium-, 4-(2-Carboxyethyliminomethyl)-1-ethylpyridinium-, 1-Benzyl-2-(4-sulfophenyliminomethyl)-pyridinium-, 1-Benzyl-4-(4-sulfophenyliminomethyl)-pyridinium-, 4-(4-Carboxyphenyliminomethyl)-1-methylpyridinium-, 2-(4-Carboxyphenyliminomethyl)-1-methylchinolinium-, 4-(4-Carboxyphenyliminomethyl)-1-methylchinolinium-, 5-(4-Carboxyphenyliminomethyl)-1-methylchinolinium-, 7-(4-Carboxyphenyliminomethyl)-1-methylchinolinium-, 2-(4-Carboxy-3-hydroxyphenyliminomethyl)-1-methylchinolinium-, 4-(4-Carboxy-3-hydroxyphenyliminomethyl)-1-methylchinolinium-, 2-(4-Cyanphenyliminomethyl)-1-methylchinolinium-, 4-(4-Cyanphenyliminomethyl)-1-methylchinolinium-, 2-(2,4-Dihydroxy-6-pyrimidyliminomethyl)-1-methylchinolinium-, 4-(2,4-Dihydroxy-6-pyrimidyliminomethyl)-1-methylchinolinium-, 4-(2,4-Dihydroxy-6-pyrimidyliminomethyl)-1-methylpyridinium-, 4-(Benzothiazol-2-yliminomethyl)-1-methylchinolinium-halogenid, wie -chlorid, -bromid oder -iodid, -benzolsulfonat, p-toluolsulfonat, C₁₋₄-alkannsulfonat, wie -methansulfonat oder -ethansulfonat, -trifluormethansulfonat, -perchlorat, 0.5 -sulfat, -hydrogensulfat, -tetrafluoroborat, -phosphat, -hexafluorophosphat oder -tetrachlorozinkat..

3. Verfahren zur Herstellung von Azomethinen mit der Formel I 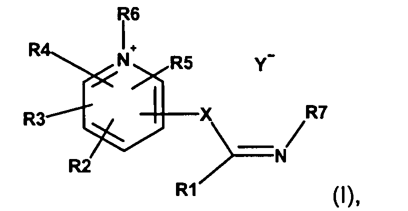 in der
• R¹ steht für ein Wasserstoffatom, eine C₁₋₄-Alkylgruppe, eine Arylgruppe oder eine Heteroarylgruppe,
• R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
• R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
• R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
• X steht für eine direkte Bindung, eine gegebenenfalls substituierte Vinylen- oder Phenylengruppe, die sich auch an einem ankondensierten aromatischen Ring befinden kann, und
• Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet,
worin eine Carbonylverbindung mit der Formel II 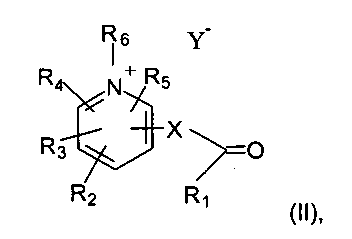 in der R¹, R², R³, R⁴, R⁵, R⁶, X und Y wie oben definiert sind,
und ein Amin mit der Formel III
H₂N-R⁷ (III),
in der R⁷ wie oben definiert ist,
in an sich bekannter Weise einer Kondensationsreaktion unterworfen werden.

4. Verfahren nach Anspruch 3, **dadurch gekennzeichnet, dass** die Verbindung mit der Formel II ausgewählt ist aus den Benzolsulfonaten, p-Toluolsulfonaten, Methansulfonaten, Perchloraten, 0.5 Sulfaten, Chloriden, Bromiden, Iodiden, Tetrachlorozinkaten von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 4-(2-Formylvinyl)-1-methylchinolium, 4-Acetyl-1-methylpyridinium, 2-Acetyl-1-methylpyridinium, 4-Acetyl-1-methylchinolin und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 4-Pyridinaldehyd-N-oxid und N-Methylpyridoxal;
2- und 4-Formyl-1-methylchinolinium-p-toluolsulfonat;
Acridin-1-carboxaldehyd, Acridin-2-carboxaldehyd, Acridin-3-carboxaldehyd, Acridin-4-carboxaldehyd, Acridin-9-carboxaldehyd, Benz[c]acridin-7-carboxaldehyd, 1-Formyl-10-methyl-acridinium-, 2-Formyl-10-methyl-acridinium-, 3-Formyl-10-methylacridinium-, 4-Formyl-10-methyl-acridinium-, 9-Formyl-10-methyl-acridinium-, 9-Formyl-2,10-dimethyl-acridinium-, 9-Formyl-10-ethyl-acridinium-, 9-Formyl-2-chlor-10-methyl-acridinium-, 9-Formyl-2-methoxy-10-methyl-acridinium-, 9-Formyl-2-chlor-10-methyl-acridinium-, 9-Formyl-3,6-dichlor-10-methyl-acridinium-, 9-Acetyl-10-methyl-acridinium-, 9-Benzolyl-10-methyl-acridinium-iodid, -bromid, -methylsulfat, -ethylsulfat -p-toluolsulfonat, -trifluormethansulfonat, -methansulfonat, -tetrafluorborat, 9-Formylacridin-N-oxid, 7-Formyl-10-methylbenz[c]acridinium-methylsulfat sowie deren beliebigen Gemischen;
Chinoliniumaldehyden und ―ketonen, insbesondere den Benzolsulfonaten, Tetrafluoroboraten, p-Toluolsulfonaten, Methansulfonaten, Trifluormethansulfonate, Perchloraten, 0.5 Sulfaten, Chloriden, Bromiden, Iodiden, Tetrafluoroboraten und/oder Tetrachlorozinkaten von 5-Formyl-1-methyl-chinolinium, 6-Formyl-1-methyl-chinolinium, 7-Formyl-1-methyl-chinolinium, 8-Formyl-1-methyl-chinolinium, 5-Formyl-1-ethyl-chinolinium, 6-Formyl-1-ethyl-chinolinium, 7-Formyl-1-ethylchinolinium, 8-Formyl-1-ethyl-chinolinium, 5-Formyl-1-benzyl-chinolinium, 6-Formyl-1-benzyl-chinolinium, 7-Formyl-1-benzyl-chinolinium, 8-Formyl-1-benzyl-chinolinium, 5-Formyl-1-allyl-chinolinium, 6-Formyl-1-allyl-chinolinium, 7-Formyl-1-allylchinolinium, 8-Formyl-1-allylchinolinium, 5-Acetyl-1-methyl-chinolinium, 6-Acetyl-1-methyl-chinolinium, 7-Acetyl-1-methyl-chinolinium, 8-Formyl-1-Acetyl-chinolinium, 9-Formyl-10-methylacridinium sowie deren beliebigen Gemischen.

5. Verfahren nach Anspruch 3 oder 4, **dadurch gekennzeichnet, dass** das Amin mit der Formel III ausgewählt ist aus 2-Hydroxyethylamin, n-Butylamin, 2-Butylamin, 2-Hydroxypropylamin, 3-Hydroxypropylamin, n-Hexylamin, Cyclohexylamin, 2,3-Dihydroxyalkylamin, 2-Aminoethansulfonsäure, α-Aminosäuren, wie Glycin, Alanin, Serin, Threonin, Lysin, Gluaminsäure, Asparaginsäure, Prolin, Tryptophan, Histidin, Arginin, Phenylalanin, 4-Hydoxyphenylalanin, β-Alanin, 6-Aminocapronsäure, Anilin, Chlor- und Brom-substitierte Aniline, Anthranilsäure, 3-Aminobenzoesäure, 4-Aminobenzoesäure, Sulfanilsäure, 4-Aminosalicysäure, 5-Aminosalicysäure, Melamin, 2-Aminobenzonitril, 3-Aminobenzonitril, 4-Aminobenzonitril, 2-Aminophenol, 3-Aminophenol, 4-Aminophenol, 4-Amino-2-aminomethylphenol, 2-Methoxyanilin, 3-Methoxyanilin, 4-Methoxyanilin, 2-Methylanilin, 3-Methylanilin, 4-Methylanilin, 4-Amino-2-methylphenol, 5-Amino-2-methylphenol, 4-Amino-3-methylphenol, 5-Amino-3-methylphenol, 4-Aminouracil, 6-Aminouracil, 5-Aminouracil, 2-Aminopyridin, 3-Aminopyridin, 4-Aminopyridin, 2-Amino-3-hydroxypyridin, 2,6-Diaminopyridin, 2,5-Diaminopyridin, 2,3-Diaminopyridin, 2-Dimethylamino-5-aminopyridin, 3-Amino-2-methylamino-6-methoxypyridin, 2,3-Diamino-6-methoxypyridin, 2,4,5-Triaminopyridin, 2,6-Dihydroxy-3,4-dimethylpyridin, 2-Hydroxy-3-aminopyridin, 2-Hydroxy-4-aminopyridin, 3,5-Diamino-2,6-dimethoxypyridin, 4,5,6-Triaminopyrimidin, 4-Hydroxy-2,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diamino-pyrazol, 3,5-Diamino-1,2,4-triazol, 3-Amino-1,2,4-triazol, 2-Aminothiazol, 5-Aminotetrazol, 3-Aminopyrazol, 3-Amino-5-hydroxypyrazol, 1-Phenyl-3-carbamoyl-4-amino-5-pyrazolon, 1-Phenyl-4,5-diamino-pyrazol, 1-Phenyl-3-methyl-4,5-diamino-pyrazol, 1-(2-Hydroxyethyl)-4,5-diamino-pyrazol. 2-Aminoimidazol, 2-Aminoimidazolin, 2-Aminobenzimidazol, 2-Aminobenzothiazol, 2-Aminobenzoxazol und/oder Sulfanilamid.

6. Verwendung von Azomethinen mit der Formel I, 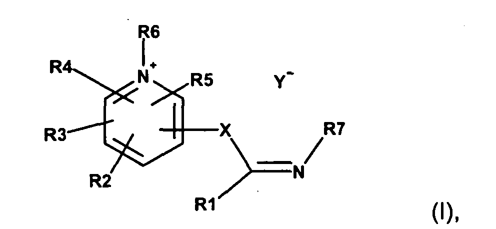 in der
• R¹ steht für ein Wasserstoffatom, eine C₁₋₄-Alkylgruppe, eine Arylgruppe oder eine Heteroarylgruppe,
• R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
• R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
• R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
• X steht für eine direkte Bindung, eine gegebenenfalls substituierte Vinylen- oder Phenylengruppe, die sich auch an einem ankondensierten aromatischen Ring befinden kann, und
• Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet,
zum Färben von keratinhaltigen Fasern.

7. Mittel zum Färben von keratinhaltigen Fasern, enthaltend mindestens ein Azomethin mit der Formel I, 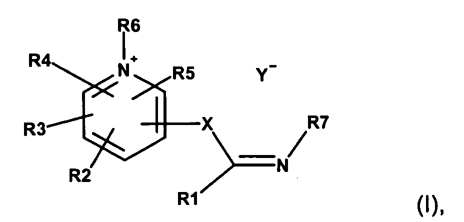 in der
• R¹ steht für ein Wasserstoffatom, eine C₁₋₄-Alkylgruppe, eine Arylgruppe oder eine Heteroarylgruppe,
• R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
• R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
• R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
• X steht für eine direkte Bindung, eine gegebenenfalls substituierte Vinylen- oder Phenylengruppe, die sich auch an einem ankondensierten aromatischen Ring befinden kann, und
• Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet.

8. Mittel nach Anspruch 7, **dadurch gekennzeichnet, dass** die Azomethine mit der Formel I in einer Menge von 0,03 bis 65 mmol, insbesondere von 1 bis 40 mmol, bezogen auf 100 g des gesamten Färbemittels, enthalten sind.

9. Mittel nach einem der Ansprüche 7 oder 8, **dadurch gekennzeichnet, dass** es als Komponente B mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, CH-aktive Verbindungen und/oder quartäre Ammoniumverbindungen enthält.

10. Mittel nach Anspruch 9, **dadurch gekennzeichnet, dass** die Komponente B ausgewählt ist aus
primären oder sekundären Aminen aus der Gruppe, bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethy)amino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest ausgewählt aus 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan, stickstoffhaltigen heterocyclischen Verbindungen ausgewählt aus der Gruppe, bestehend aus 2-Aminopyridin, 3-Aminopyridin, 4-Aminopyridin, 2-Amino-3-hydroxypyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und Hydroxypyrimidine, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxyphenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure,
CH-aktiven Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolinhydrochlorid, 3-Cyan-1,4-dimethyl-6-hydroxy-2-pyridon und 3-Cyan-1-ethyl-6-hydroxy-4-methyl-2-pyridon,
quartäre Ammoniumsalze ausgewählt aus der Gruppe Tetramethyl-, Tetraethyl-, Tetrapropyl-, Tetrabutyl-, Benzyltrimethylammonium-chlorid, -bromid, -iodid, -hydrogensulfat, 0.5 -sulfat sowie den quaternierten Hydroxyethylcellulose-Derivaten (INCI-Bezeichnung: Polyquaternium 10).

11. Mittel nach Anspruch 10, **dadurch gekennzeichnet, dass** die Komponente B ausgewählt ist aus der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat sowie deren vorzugsweise mit anorganischen Säuren gebildeten physiologisch verträglichen Salzen.

12. Mittel nach einem der Ansprüche 7 bis 11, **dadurch gekennzeichnet, dass** es als zusätzliche Komponente C reaktive Carbonylverbindungen, insbesondere Aldehyde und/oder cyclische oder nichtcyclische Ketone, enthält.

13. Mittel nach einem der Ansprüche 7 bis 12, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

14. Mittel nach einem der Ansprüche 7 bis 13, **dadurch gekennzeichnet, dass** es direktziehende Farbstoffe aus der Gruppe der Nitrophenylendiamine, Nitroaminöphenole, Anthrachinone oder Indophenole vorzugsweise in einer Menge von 0,01 bis 20 Gew.-%, bezogen auf das gesamte Färbemittel, enthält.

15. Mittel nach einem der Ansprüche 7 bis 14, **dadurch gekennzeichnet, dass** es anionische, zwitterionische oder nichtionische Tenside enthält.

16. Mittel nach einem der Ansprüche 7 bis 15, **dadurch gekennzeichnet, dass** es Oxidationsmittel, insbesondere H₂O₂, in einer Menge von 0,01 bis 6 Gew.-%, bezogen auf die Anwendungslösung, enthält.

17. Mittel nach einem der Ansprüche 7 bis 16, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Ammonium, Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, enthalten sind.

18. Verfahren zum Färben von keratinhaltigen Fasern, insbesondere menschlichen Haaren, worin ein Färbemittel, enthaltend mindestens ein Azomethin mit der Formel I 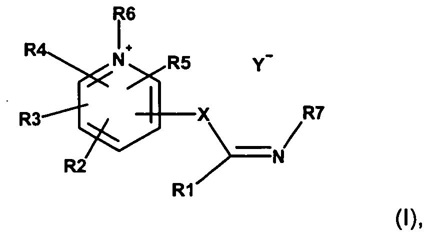 in der
• R¹ steht für ein Wasserstoffatom, eine C₁₋₄-Alkylgruppe, eine Arylgruppe oder eine Heteroarylgruppe,
• R², R³, R⁴ und R⁵ unabhängig voneinander stehen für ein Wasserstoffatom, ein Halogenatom, eine C₁₋₄-Alkylgruppe, eine C₁₋₄-Alkoxygruppe, eine C₁₋₄-Hydroxyalkoxygruppe, eine Hydroxygruppe, eine Nitrogruppe, eine Cyanogruppe oder eine Aminogruppe, die durch C₁₋₄-Alkylgruppen substituiert sein kann, oder eine C₁₋₄-Acylgruppe,
wobei auch jeweils zwei der Reste R², R³, R⁴, und R⁵ einen oder zwei an das Restmolekül ankondensierte aromatische Ringe bilden oder einer der Reste R², R³, R⁴ oder R⁵ gemeinsam mit der Gruppe -X-C(=NR7)R¹- einen ankondensierten 5-, 6- oder 7-Ring bilden können,
• R⁶ steht für eine C₁₋₄-Alkylgruppe, eine C₁₋₄-Carboxylatoalkylgruppe, eine C₁₋₄-Sulfonatoalkylgruppe, eine Arylgruppe, eine Aryl-C₁₋₄-alkyl-gruppe, eine Heteroarylgruppe oder ein negativ geladenes Sauerstoffatom,
• R⁷ steht für eine C₁₋₆-Alkylgruppe, eine C₁₋₄-Hydroxyalkylgruppe, eine C₃₋₆-Cycloalkylgruppe, eine C₂₋₄-Carboxyalkylgruppe, eine C₁₋₄-Sulfoalkylgruppe, einen substituierten C₁₋₆-Alkylrest, dessen Substitutionsmuster sich von α-Aminosäuren ableitet,
oder eine Aryl-, Aryl-C₁₋₄-alkyl- oder eine 5- oder 6- gliedrige heterocyclische-Gruppe, welche gegebenenfalls durch Halogen, C₁₋₄-Alkyl-, C₁₋₄-Alkoxy-, Cyan-, Amino-, Alkylamino-, Hydroxy-, Sulfo-, Sulfamoyl-, Carboxy- oder Carbonamidgruppen substituiert sein können,
• X steht für eine direkte Bindung, eine gegebenenfalls substituierte Vinylen- oder Phenylengruppe, die sich auch an einem ankondensierten aromatischen Ring befinden kann, und
• Y⁻ steht für ein Halogenid-, wie Chlorid, Bromid oder Iodid, ein Benzolsulfonat-, ein p-Toluolsulfonat-, ein C₁₋₄-Alkansulfonat-, wie Methansulfonat oder Ethansulfonat, Trifluormethansulfonat, ein Perchlorat-, ein 0.5 Sulfat-, ein Hydrogensulfat-, ein Tetrafluoroborat-, ein 0.3 Phosphat-, ein Hexafluorophosphat- oder ein Tetrachlorozinkation, wobei Y⁻ entfällt, wenn R⁶ eine negativ geladene Gruppe ist, die mit dem positiv geladenen Stickstoffatom, an dem sie gebunden ist, ein Zwitterion bildet,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.
